# EUROPÄISCHE PATENTSCHRIFT

(11) **EP 2 768 800 B1**
(45) Veröffentlichungstag und Bekanntmachung des Hinweises auf die Patenterteilung: **20.01.2016**
(21) Anmeldenummer: 12775665.8
(22) Anmeldetag: 18.10.2012
(51) Int. Cl.: C07C 263/10, C07C 265/14

(54) **VERFAHREN ZUR HERSTELLUNG FARBHELLER POLYISOCYANATE**
PROCESS FOR THE PREPARATION OF LIGHT-COLOURED POLYISOCYANATES
PROCÉDÉ POUR LA PRÉPARATION DE POLYISOCYANATES PEU COLORÉS

(30) Priorität: 21.10.2011 DE 102011084965
(43) Veröffentlichungstag der Anmeldung: 27.08.2014
(73) Patentinhaber: Covestro Deutschland AG, 51373 Leverkusen (DE)
(72) Erfinder: MUELLER, Thomas, Ernst, 52062 Aachen (DE); GUERTLER, Christoph, 50735 (DE); SCHERER-WERKE, Axel, 25524 Itzehoe (DE); WERSHOFEN, Stefan, 41065 Mönchengladbach (DE); VOGT, Henning, 52062 Aachen (DE); LEITNER, Walter, 52074 Aachen (DE)
(74) Vertreter: Levpat
(86) Internationale Anmeldenummer: PCT/EP2012/070613
(87) Internationale Veröffentlichungsnummer: WO 2013/057165

(56) Entgegenhaltungen:
- EP-A1- 0 581 100
- EP-A1- 2 103 595
- EP-A2- 0 866 057

## Beschreibung

Die Erfindung betrifft ein Verfahren zur Herstellung von farbhellen Polyisocyanaten durch Phosgenierung von Mischungen enthaltend MDA und mehrkernige aromatische Polyamine, die über Methylenbrücken eingebaute *o*-Phenylendiamin-Einheiten aufweisen. Die Erfindung betrifft weiterhin die aus diesem Verfahren erhältlichen Polyisocyanatgemische.

Unter Di- und Polyaminen der Diphenylmethanreihe (MDA) werden Amine und Gemische von Aminen entsprechend der Formel (I) verstanden: 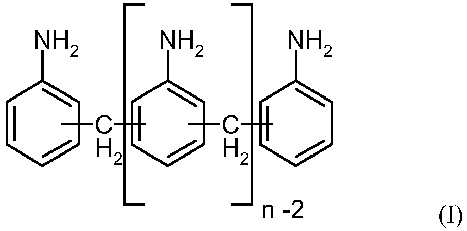 wobei n für eine natürliche Zahl ≥ 2 steht.

Für Verbindungen und Verbindungsgemische mit n = 2 sind dabei auch die Bezeichnung monomeres MDA (MMDA) und für Verbindungen und Verbindungsgemische mit n > 2 auch die Bezeichnung polymeres MDA (PMDA) üblich. Verbindungsgemische, in denen Verbindungen mit n = 2 und n > 2 nebeneinander vorkommen, werden üblicherweise vereinfachend unter dem Begriff MDA (Di- und Polyamine der Diphenylmethanreihe) zusammengefasst.

Isocyanate und Isocyanatgemische werden gemäß dem Stand der Technik durch Phosgenierung der entsprechenden Amine hergestellt. Für Polyurethan(PUR)-Schäume finden beispielsweise di- oder polyfunktionelle aromatische Isocyanate der Diphenylmethan-Reihe (MDI) Anwendung. Bedingt durch den Herstellungsprozess werden nach der Phosgenierung und der anschließenden Aufarbeitung (Abtrennung des Lösemittels; Abtrennen von monomerem MDI) oft dunkel gefärbte Produkte erhalten, die wiederum verfärbte PolyurethanSchäume oder andere, ebenfalls verfärbte Polyurethan-Materialien ergeben. Dies ist unerwünscht, da eine solche Färbung den visuellen Gesamteindruck beeinträchtigt und geringfügige Inhomogenitäten hervortreten lässt, z.B. als Schlieren in den erhaltenen Schäumen. Helle Isocyanate, bzw. Isocyanate, die eine reduzierte Menge an farbgebenden Komponenten enthalten, werden deshalb als Rohstoffe bevorzugt.

Es hat daher nicht an Versuchen gefehlt, Isocyanate und insbesondere die Di- und Polyisocyanate der Diphenylmethan-Reihe (MDI) mit heller Farbe zu erhalten. Zur empirischen Farbaufhellung von MDI sind zahlreiche Methoden bekannt. Die Natur der störenden Farbkörper (farbgebende Moleküle, farbgebende Aggregate und/oder farbgebende Partikel) ist bisher jedoch nur unzureichend geklärt.

Die bislang bekannten Verfahren lassen sich in fünf Gruppen unterteilen, die im Folgenden anhand der aufgeführten Zitate beispielhaft dargestellt werden:
1. Verfahren, bei denen für die Herstellung von MDA Anilin eines bestimmten Reinheitsgrades eingesetzt wird.
   EP-A1 1 813 598 lehrt, dass sich der Einsatz von Anilin, das weniger als 3 Gew.-%, bevorzugt 0,001 bis 3 Gew.-%, besonders bevorzugt 0,01 bis 1 Gew.-% Di- und Polyamine der Diphenylmethanreihe, bezogen auf das Gewicht des eingesetzten Anilins, enthält, bei der Herstellung von MDA vorteilhaft auf die Farbe des daraus durch Phosgenierung hergestellten MDI auswirkt. Die im Anilin enthaltenen Di- und Polyamine der Diphenylmethanreihe gelangen in das zur Herstellung von MDA eingesetzte Amin, da üblicherweise bei der MDA-Herstellung Anilin im Überschuss eingesetzt wird, und der Überschuss nach z.B. destillativer Abtrennung vom Produkt wieder in den Prozess zurückgeführt wird.
   Weitere im Anilin enthaltene Nebenkomponenten sind z.B. in RD 510004 (Research Disclosure Journal, Veröffentlichung Oktober 2006) aufgeführt, so z.B. Cyclohexylamin, Cyclohexanol, Cyclohexanon, Phenol usw., ohne dass erwähnt wird, in welchen Gehalten diese Nebenkomponenten im Anilin enthalten sind und ob diese Nebenkomponenten Einfluss auf die Qualität des MDAs und des daraus gewonnenen MDIs haben.
   EP-A1 2 103 595 beschreibt ein Verfahren zur Herstellung von Di- und Polyaminen der Diphenylmethanreihe (MDA) durch Umsetzung von Anilin und Formaldehyd in Gegenwart eines sauren Katalysators, bei dem das eingesetzte Anilin in der Summe weniger als 0,5 Gew.-%, bevorzugt 0,0001 bis 0,4 Gew.-%, besonders bevorzugt 0,0001 bis 0,3 Gew.-%, und insbesondere bevorzugt 0,0001 bis 0,25 Gew.-%, bezogen auf das Gewicht des eingesetzten Anilins, an Verbindungen enthält, die wenigstens eine Carbonylgruppe enthalten oder die durch Reaktion dieser wenigstens eine Carbonylgruppe enthaltenden Verbindungen mit Anilin entstehen.
2. Verfahren, bei denen die als Ausgangsmaterial eingesetzten Di- und Polyamine der Diphenylmethanreihe (MDA) einer Behandlung und/oder Reinigung unterzogen wurden.
   Die EP-A 0 546 398 beschreibt ein Verfahren zur Herstellung von MDI, bei dem das als Edukt verwendete MDA vor der Phosgenierung angesäuert wird.
   Die EP-A 0 446 781 betrifft ein Verfahren zur Herstellung von MDI, bei dem das MDA zuerst mit Wasserstoff behandelt und anschließend einer Phosgenierung unterzogen wird, wobei ein helleres MDI erhalten wird.
   Die oben genannten Verfahren ergeben nur eine geringfügige Farbverbesserung, da die Farbkörper im MDI erfahrungsgemäß nicht nur aus bestimmten Nebenkomponenten der MDA-Herstellung bestehen, sondern auch aus Farbprecursoren resultieren, die durch Nebenreaktionen während der Phosgenierung gebildet werden.
3. Verfahrenstechnische Lösungen im Phosgenierungsprozess
   Die US-A 5 364 958 betrifft ein Verfahren zur Herstellung von Isocyanaten, bei dem nach der Phosgenierung das Phosgen bei niedriger Temperatur vollständig entfernt wird und anschließend das Isocyanat in der Wärme mit HCl-Gas behandelt wird.
   In der DE-A-1981 7691 wird ein Verfahren zur Herstellung von MDI mit vermindertem Gehalt an chlorierten Nebenprodukten und verminderter Iodfarbzahl durch Einhaltung definierter Parameter in der Phosgenierungsreaktion beschrieben. Insbesondere ist hier die Einhaltung bestimmter Phosgen/HCl-Verhältnisse in der Reaktionsstufe erforderlich. Dieses Verfahren hat den Nachteil, dass eine Variation der Parameter in der Phosgenierung erschwert und dadurch die Qualität der Phosgenierung sehr anfällig wird. Durch die fehlende Flexibilität der Parameter in der Phosgenierung wird außerdem eine praktische Durchführung der Phosgenierung sehr schwierig und erfordert einen hohen technischen Aufwand.
   Eine andere Möglichkeit der Verbesserung der Farbe von Isocyanaten ist gemäß EP-B 1 187 808 die Verwendung von Phosgen mit niedrigen Gehalten an Brom- und/oder Jod.
   EP-B 1 808 430 beschreibt eine Verbesserung der Farbe von Isocyanaten durch den Einsatz von Phosgen, das nur einen geringen Gehalt an Schwefel in elementarer oder gebundener Form aufweist, in der Phosgenierung.
   Verfahren der genannten Art versuchen zwar, die Verfärbungen verursachenden Komponenten an der richtigen Stelle abzutrennen, sie sind jedoch sowohl aufgrund ihres hohen technischen Aufwands als auch in ihrem Farbaufhellungseffekt zu wenig effizient, da der Abbau von Farbvorläufern, die durch unvollständig ablaufende chemische Reaktionen entstehen, nur in geringem Umfang erfolgt.
4. Zusatz von farbaufhellenden Additiven zum Isocyanat-Rohprodukt nach erfolgter Phosgenierung und vor der Aufarbeitung z.B. durch Destillation.
   Die EP-A 0 581 100 betrifft ein Verfahren zur Herstellung von Isocyanaten, bei dem nach der Phosgenierung ein chemisches Reduktionsmittel vor dem Abtrennen von Lösemittel zugegeben wird, wobei gemäß dieser Druckschrift ebenfalls helle Produkte erhalten werden.
   Gemäß der US-A 4 465 639 wird dem nach der Phosgenierung erhaltenen Rohprodukt zur Farbaufhellung Wasser zugesetzt. Zum gleichen Zweck beschreiben die EP-A 538 500, EP-A 0 445 602 und EP-A 0 467 125 den Zusatz von Carbonsäuren, Alkanolen bzw. Polyetherpolyolen.
   Die oben beschriebenen Verfahren zur Farbaufhellung sind zwar effizient, sie weisen jedoch Nachteile dahingehend auf, dass neben der Farbaufhellung die zugegebenen Additive Reaktionen mit den als Produkt erhaltenen Isocyanaten eingehen und daraus beispielsweise eine unerwünschte Verminderung des Isocyanatgehalts resultiert. Außerdem besteht die Gefahr der Bildung von unerwünschten Nebenprodukten im MDI.
5. Nachbehandlung des Endprodukts
   Die EP-A 0 133 538 beschreibt die Reinigung von Isocyanaten durch Extraktion, wodurch Fraktionen eines hellen MDI erhalten werden.
   Die EP-A 0 561 225 betrifft ein Verfahren zur Herstellung von Isocyanaten oder Isocyanatgemischen, die laut dieser Schrift keine farbgebenden Komponenten aufweisen, wobei die Isocyanate nach der Phosgenierung der entsprechenden Amine einer Wasserstoffbehandlung bei einem Druck von 1 bis 150 bar und einer Temperatur von 100 bis 180 °C unterworfen werden. Dabei werden gemäß den dort beschriebenen Beispielen Isocyanat-Endprodukte als solche oder in Form ihrer Lösungen in geeigneten Lösemitteln hydriert.
   Diese farbverbessernden Nachbehandlungen der Isocyanat-Endprodukte nach der vollständigen Abtrennung des Lösemittels bei erhöhter Temperatur sind ebenfalls wenig effizient, da durch die hohen Temperaturen, die bei der Aufarbeitung, insbesondere dem Abdestillieren des Lösemittels und ggf. von monomerem MDI (Diisocyanate) auftreten, bereits stabile Farbkörper entstanden sind, die chemisch nur noch schwer abzubauen sind.

EP 0 866 057 offenbart ein Verfahren zur Herstellung von farbhellen Isocyanaten wie MDI durch Umsetzung der entsprechenden Amine mit Phosgen. Die hellere Farbe wird dadurch erzielt, dass die Amine vor der Phosgenierung mit festen anorganischen Substanzen, die lewissaure und/oder brönstedtsaure Zentren enthalten, behandelt werden.

Die Aufgabe der vorliegenden Erfindung bestand darin, ein einfaches und wirtschaftliches Verfahren zur Herstellung von hellen Polyisocyanaten zur Verfügung zu stellen. Dabei soll das Verfahren ohne die oben genannten Behandlungsschritte auskommen und dennoch zu hellen Isocyanaten, die zur Herstellung von Polyurethanen oder deren Vorläufern (z.B. Prepolymeren) geeignet sind, führen.
Überraschenderweise wurde gefunden, dass durch Phosgenierung von Mischungen enthaltend MDA und mehrkernige aromatische Polyamine, die über Methylenbrücken eingebaute *o-*Phenylendiamin-Einheiten aufweisen, Polyisocyanatgemische erhalten werden, die gegenüber Polyisocyanaten auf Basis von MDI allein bei etwa gleichem Gelbwert einen deutlich verringerten Grauwert aufweisen.
Gegenstand der Erfindung ist daher ein Verfahren zur Herstellung von farbhellen Polyisocyanatgemischen durch Phosgenierung von Mischungen enthaltend MDA und mehrkernige aromatische Polyamine der Formel (II) und/oder (III) 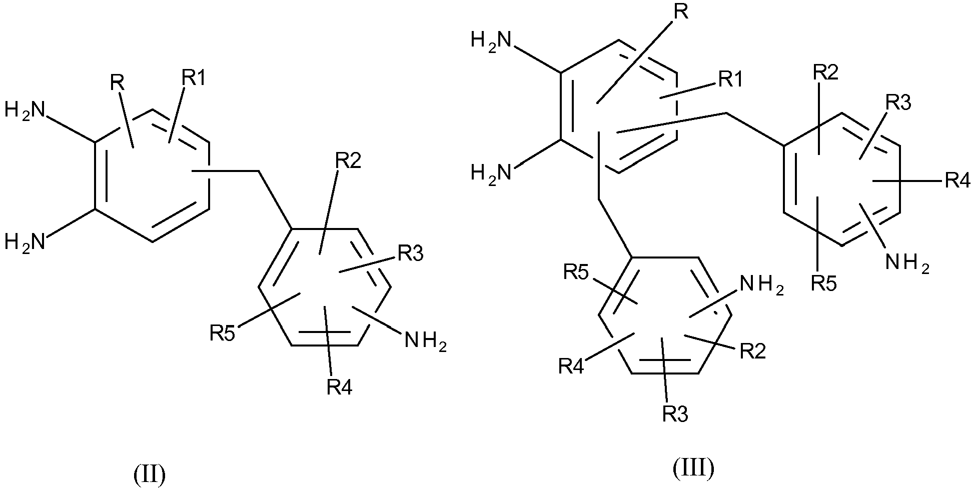 und/oder höhere Homologe der Verbindungen der Formel (II) und/oder (III), wobei
R für Wasserstoff, einen gegebenenfalls substituierten oder Heteroatome enthaltenden gesättigten oder ungesättigten Alkyl-, Cycloalkyl-, Aralkyl- oder Arylrest und
R1 für Wasserstoff, einen gegebenenfalls substituierten oder Heteroatome enthaltenden gesättigten oder ungesättigten Alkyl-, Cycloalkyl-, Aralkyl- oder Arylrest steht,
oder R und R1 direkt miteinander verknüpft sind, so dass sie zusammen mit der Phenylendiaminteilstruktur ein bicyclisches Strukturelement bilden, und
R2, R3, R4 und R5 unabhängig voneinander für Wasserstoff oder einen ggf. substituierten oder Heteroatome enthaltenden gesättigten oder ungesättigten Alkyl-, Cycloalkyl-, Aralkyl-oder Arylrest in *ortho-, meta*- oder *para-*Stellung zur an den gleichen aromatischen Ring gebundenen Aminogruppe stehen.

Die Reste R2, R3, R4, R5 können auch teilweise oder alle direkt miteinander verknüpft sein, so dass sie zusammen mit dem an die Aminogruppe gebundenen aromatischen System bi-, tri-oder tetracyclische Systeme bilden.

R und R1 stehen unabhängig voneinander für (a) Wasserstoff, (b) einen gesättigten oder ungesättigten Alkylrest wie z.B. Methyl, Ethyl, Vinyl, Propyl, Allyl, Isopropyl, die verschiedenen isomeren Butylreste, die verschiedenen isomeren Pentylreste, die verschiedenen isomeren Hexylreste, lineare oder verzweigte Alkylreste mit mehr als 6 C-Atomen, (c) einen gesättigten oder ungesättigten Cycloalkylrest wie z.B. Cyclopropyl, Cyclobutyl, Cyclopentyl, Cyclopentenyl, Cyclopentadienyl, Cyclohexyl, (d) einen gesättigten oder ungesättigten Aralkylrest wie z.B. Benzyl, 2-Phenylvinyl oder (e) einen Arylrest wie z.B. Phenyl. Die genannten Reste R und R1 können ggf. auch weitere Substituenten tragen und ggf. von Kohlenstoff und Wasserstoff verschiedene Atome (Heteroatome) enthalten, wie z.B. Sauerstoff, Stickstoff, Fluor, Chlor, Brom.

Vorzugsweise stehen R und R1 unabhängig voneinander für Wasserstoff oder Methyl.

Bevorzugt sind die Reste R2 bis R5 Wasserstoff.

Besonders bevorzugt leitet sich der Rest (H₂N)₂C₆HRR1 in Formel (II) bzw. (H₂N)₂C₆RR1 in Formel (III) von o-Phenylendiamin, 1,2-Diamino-3-methylbenzol (2,3-TDA) oder 1,2-Diamino-4-methylbenzol (3,4-TDA), ganz besonders bevorzugt von 1,2-Diamino-3-methylbenzol oder 1,2-Diamino-4-methylbenzol ab.

Es können auch Gemische enthaltend verschiedene Verbindungen der Formel (II) und/oder (III) und/oder der höheren Homologe der Formel (II) und/oder (III), die sich in Art und Position der Reste R, R1, R2, R3, R4 und R5 unterscheiden, eingesetzt werden.

Als höhere Homologe im Sinne der Erfindung werden Verbindungen bezeichnet, bei denen beliebig viele Verbindungen der Formel (IV), im folgenden allgemein auch o-Phenylendiamine genannt, 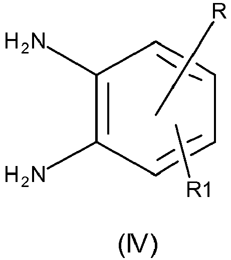 und/oder der Formel (V) 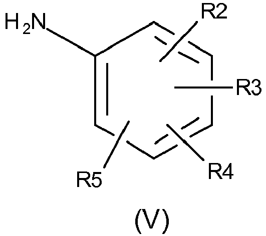 wobei R und R1 bis R5 die oben angegebene Bedeutung haben,
an einem oder mehreren der aromatischen Kohlenstoffatome über Methylengruppen zu linearen oder verzweigten Ketten oder zyklischen Strukturen verknüpft sind, wobei mindestens ein o-Phenylendiamin (Verbindung der Formel (IV)) im Molekül enthalten ist. Der Gehalt an mehrkernigen aromatischen Polyaminen (II) und (III) und/oder deren höheren Homologen in den Ausgangsmischungen beträgt 0,001 bis 5, bevorzugt 0,001 bis 3,5, besonders bevorzugt 0,001 bis 2 Gewichtsprozent bezogen auf das Gesamtgewicht aus MDA, den mehrkernigen aromatischen Polyaminen (II) und (III) und deren höheren Homologen.

Insbesondere beträgt der Gehalt der mehrkernigen aromatischen Polyamine (II) und (III) in den Ausgangsmischungen 0,001 bis 2, bevorzugt 0,001 bis 1, besonders bevorzugt 0,001 bis 0,5 Gewichtsprozent bezogen auf das Gesamtgewicht aus MDA, den mehrkernigen aromatischen Polyaminen (II) und (III) und deren höheren Homologen. Die genaue Definition der Angabe Gewichtsprozent kann der Beschreibung der Analysenmethode weiter unten im experimentellen Teil entnommen werden.

Ein weiterer Gegenstand der Erfindung sind Polyisocyanatmischungen enthaltend MDI und mehrkernige aromatische Polyisocyanate, die von den genannten mehrkernigen aromatischen Polyaminen (II) und/oder (III), sowie deren höheren Homologen abgeleitet sind, wobei R und R1 bis R5 die oben angegebene Bedeutung haben.

Abgeleitet im Sinne der Erfindung bedeutet, dass die Aminogruppen der Polyamine (II) bzw. (III), bzw. deren höherer Homologe, ganz oder teilweise durch Isocyanatgruppen ersetzt werden.

Bei der Phosgenierung der Polyamine (II) bzw. (III) bzw. deren höherer Homologe können die enthaltenen *o*-Phenylendiamin-Einheiten auch zu den entsprechenden zyklischen Harnstoffen umgesetzt werden.

Der Gehalt dieser mehrkernigen aromatischen Polyisocyanate, die von den genannten mehrkernigen aromatischen Polyaminen (II) und (III), sowie deren höheren Homologen abgeleitet sind, in den beanspruchten Polyisocyanatmischungen beträgt 0,001 bis 5, bevorzugt 0,001 bis 3,5, besonders bevorzugt 0,001 bis 2 Gewichtsprozent bezogen auf das Gesamtgewicht aus MDI und den mehrkernigen aromatischen Polyisocyanaten, die von den genannten mehrkernigen aromatischen Polyaminen (II) und (III), sowie deren höheren Homologen abgeleitet sind.

Insbesondere beträgt der Gehalt der mehrkernigen aromatischen Polyisocyanate, die von den genannten mehrkernigen aromatischen Polyaminen (II) und (III) in den Ausgangsmischungen abgeleitet sind, 0,001 bis 2, bevorzugt 0,001 bis 1, besonders bevorzugt 0,001 bis 0,5 Gewichtsprozent bezogen auf das Gesamtgewicht aus MDI und den mehrkernigen aromatischen Polyisocyanaten, die von den mehrkernigen aromatischen Polyaminen (II) und (III) sowie deren höheren Homologen abgeleitet sind.

Die in das erfindungsgemäße Verfahren einzusetzenden MDA/Polyamin-Gemische sind beispielsweise erhältlich durch Co-Kondensation von *o*-Phenylendiaminen (Formel IV) und Anilin oder einem Gemisch enthaltend Anilin und ein oder mehrere primäre aromatische Monoamine der Formel V, bei denen mindestens ein in *ortho-* oder *para-*Stellung zur Aminogruppe befindliches Kohlenstoffatom einen Wasserstoffsubstituenten trägt, mit Formaldehyd, wobei die Menge an eingesetzten o-Phenylendiaminen 0,001 bis 2, bevorzugt 0,001 bis 1,5, besonders bevorzugt 0,001 bis 0,9 Gewichtsprozent bezogen auf das Gewicht des eingesetzten Anilins und der eingesetzten Monoamine beträgt.

Dabei kann die Co-Kondensation so durchgeführt werden, dass Anilin bzw. das Gemisch enthaltend Anilin und ein oder mehrere primäre aromatische Monoamine der Formel V und Formaldehyd mit protonensauren Katalysatoren zu Intermediaten vorkondensiert werden und dann, entweder zur Reaktionsmischung oder zu isolierten Intermediaten, die *o-*Phenylendiamine zugegeben werden.

Formaldehyd wird technisch üblicherweise als wässrige Lösung eingesetzt, die in Konzentrationen von 30-50 Gew.-% vorliegt. Es ist aber auch möglich, wässrige Formaldehydlösungen anderer Konzentration, gasförmiges Formaldehyd, Lösungen von Formaldehyd in anderen Lösungsmittels als Wasser oder andere Methylengruppen liefernde Verbindungen, wie z.B. Polyoxymethylenglykol, Paraformaldehyd oder Trioxan einzusetzen. Die wässrigen Formaldehydlösungen können auch Methanol, üblicherweise im Konzentrationsbereich 0,001 - 15 Gew.-% bezogen auf die wässrige Formaldehydlösung, und/oder Ameisensäure, üblicherweise im Konzentrationsbereich 0,0001 - 0,1 Gew.-%, bevorzugt 0,0001 - 0,03 Gew.-% bezogen auf die wässrige Formaldehydlösung, enthalten.

Protonensäuren sind alle Protonensäure mit einem pKₐ-Wert kleiner als 3, beispielsweise Salzsäure, (wässrige) HBr, (wässrige) HI, Schwefelsäure, *p*-Toluolsulfonsäure, Methansulfonsäure, Phosphorsäure, Perchlorsäure, Trichloressigsäure, Trifluoressigsäure, Trifluormethansulfonsäure, saure Ionenaustauscher mit Sulfonsäuregruppen sowie feste Säure wie Aluminosilikate, Zeolithe oder mesoporöse Aluminosilikate. Eine bevorzugte Säure ist Salzsäure.

Üblicherweise wird kein zusätzliches Lösungsmittel für die Umsetzung verwendet; überschüssiges Anilin oder überschüssiges Gemisch enthaltend Anilin und ein oder mehrere primäre aromatische Monoamine der Formel V dient als Lösungsmittel. Allerdings ist es möglich, die Reaktion in Gegenwart eines zusätzlichen Lösungsmittels oder Gemischen mehrerer zusätzlicher Löungsmittel durchzuführen. Geeignete Lösungsmittel sind z.B. Wasser, Alkohole wie Methanol, Ethanol, die isomeren Propanole, die isomeren Butanole, höhere Alkohole, Chlorbenzol, Dichlorbenzol, Dimethylformamid, Dimethylacetamid, Dimethylsulfoxid, *N*-Methylpyrrolidon, 1,4-Dioxan, Tetrahydrofuran, Glycol und dessen höhere Homologe H(O-CH₂-CH₂-)ₙOH (n≥2).

Anilin bzw. ein Gemisch enthaltend Anilin und ein oder mehrere primäre aromatische Monoamine der Formel V und Formaldehyd werden in einem molaren Verhältnis 1,5:1 bis 10:1, bevorzugt 1,5:1 bis 6:1, besonders bevorzugt 1,8:1 bis 4:1 und ganz besonders bevorzugt 1,8:1 bis 2,5:1 eingesetzt. Das molare Verhältnis der Protonensäure, bevorzugt Salzsäure, zum Anilin bzw. zum Gemisch enthaltend Anilin und ein oder mehrere primäre aromatische Monoamine der Formel V beträgt üblicherweise 1:100 bis 1:1, bevorzugt 1:100 bis 1:2, besonders bevorzugt 1:50 bis 1:5 und ganz besonders bevorzugt 1:20 bis 1:10.

Das Verfahren kann z.B. so durchgeführt werden, dass Anilin bzw. das Gemisch enthaltend Anilin und ein oder mehrere primäre aromatische Monoamine der Formel V, Formaldehyd-Lösung und Salzsäure in einen Rührkessel gegeben und vermischt werden und ggf. parallel zu der stattfindenden Umsetzung ein Teil des Wassers durch destillative Abtrennung entfernt wird. Gegebenenfalls können Anilin bzw. das Gemisch enthaltend Anilin und ein oder mehrere primäre aromatische Monoamine der Formel V, die Formaldehyd-Lösung und Salzsäure bei einem diskontinuierlichen Verfahren auch über zeitliche Dosierprofile zugegeben werden, wobei die Wasserabtrennung während oder nach der Zugabe der Edukte, beispielsweise mittels Vakuumdestillation, stattfinden kann. Die Vermischung von Anilin bzw. dem Gemisch enthaltend Anilin und ein oder mehrere primäre aromatische Monoamine der Formel V, Formaldehyd-Lösung und wässriger HCl findet bei Temperaturen von 0 bis 80 °C, bevorzugt 20 bis 60 °C und besonders bevorzugt 30 bis 40 °C statt.

Das Verfahren kann auch so durchgeführt werden, dass Anilin bzw. das Gemisch enthaltend Anilin und ein oder mehrere primäre aromatische Monoamine der Formel V und Formaldehyd in Abwesenheit des sauren Katalysators bei Temperaturen von 20 °C bis 100 °C, bevorzugt von 40 °C bis 100 °C, besonders bevorzugt von 60 °C bis 95 °C vermischt und zur Reaktion gebracht werden. Hierbei bilden sich Kondensationsprodukte aus Anilin bzw. Anilin und den eingesetzten primären aromatischen Monoaminen der Formel V und Formaldehyd (sogenanntes Aminal). Im Anschluss an die Aminalbildung wird das in dem Aminal enthaltene Wasser beispielsweise durch Phasentrennung oder durch andere geeignete Verfahren, beispielsweise durch Destillation, zumindest teilweise entfernt. Die Zugabe des sauren Katalysators und die Entfernung des Wassers können beispielsweise so erfolgen, dass in einen das erzeugte Aminal enthaltenden Rührkessel wässrige HCl zugegeben wird und ggf. ein Teil des Wassers während der Umsetzung zum Kondensationsprodukt durch destillative Abtrennung entfernt wird.

In einer besonderen Variante dieser Ausführungsform werden zunächst Anilin bzw. das Gemisch enthaltend Anilin und ein oder mehrere primäre aromatische Monoamine der Formel V und Formaldehyd in Abwesenheit des sauren Katalysators bei Temperaturen von 20 °C bis 100 °C, bevorzugt von 40 °C bis 100 °C, besonders bevorzugt von 60 °C bis 95 °C vermischt und zur Reaktion gebracht. Hierbei bilden sich Kondensationsprodukte aus Anilin bzw. Anilin und den eingesetzten primären aromatischen Monoaminen der Formel V und Formaldehyd (sogenanntes Aminal). Im Anschluss an die Aminalbildung wird das in dem Aminal enthaltene Wasser beispielsweise durch Phasentrennung oder durch andere geeignete Verfahren, beispielsweise durch Destillation, zumindest teilweise entfernt. Das Aminal wird dann mit Salzsäure bei Temperaturen von 0 bis 80 °C, bevorzugt 20 bis 60 °C und besonders bevorzugt 30 bis 40 °C vermischt.

Zu dem nach einer der vorstehenden Ausführungsformen erhaltenen Reaktionsgemisch wird das o-Phenylendiamin der Formel (IV) oder ein Gemisch aus zwei oder mehr o-Phenylendiaminen der allgemeinen Formel (IV), wobei die Bestandteile des genannten Gemisches in beliebigen Mengenverhältnissen vorliegen können, bei Temperaturen von 0 bis 100 °C, bevorzugt 20 bis 80 °C und besonders bevorzugt 60 bis 80 °C zugeführt.

Die Zugabe des o-Phenylendiamins oder eines Gemisches aus zwei oder mehr o-Phenylendiaminen erfolgt in Substanz (als Feststoff oder als Flüssigkeit bzw. Schmelze) oder als Lösung in einem geeigneten Lösungsmittel. Geeignete Lösungsmittel sind z.B. Wasser, Alkohole wie Methanol, Ethanol, die isomeren Propanole, die isomeren Butanole, höhere Alkohole, Chlorbenzol, Dichlorbenzol, Dimethylformamid, Dimethylacetamid, Dimethylsulfoxid, *N*-Methylpyrrolidon, 1,4-Dioxan, Tetrahydrofuran, Glycol und dessen höhere Homologe H(O-CH₂-CH₂-)ₙOH (n≥2), primäre aromatische Monoamine der Formel (V), wie z.B. Anilin, oder Gemische derselben.

Bezogen auf 1 Mol Anilin bzw. 1 Mol des Gemisches enthaltend Anilin und ein oder mehrere primäre Monoamine der Formel V werden üblicherweise 0,000 01 - 0,02 Mol des o-Phenylendiamins der Formel (IV) oder eines Gemisches bestehend aus zwei oder mehr o-Phenylendiaminen der Formel (IV), bevorzugt 0,000 01 - 0,015 Mol, besonders bevorzugt 0,000 01 - 0,009 Mol, eingesetzt.

Der Zeitpunkt der Zugabe des *o*-Phenylendiamins der Formel (IV) oder eines Gemisches bestehend aus zwei oder mehr *o*-Phenylendiaminen der allgemeinen Formel (IV) wird so gewählt, dass der Formaldehyd praktisch vollständig umgesetzt ist und die gebildeten Methylengruppen von weniger als zwei Heteroatomen substituiert sind. Dadurch wird die Bildung von Nebenkomponenten mit Benzimidazolstruktur weitgehend vermieden.

In Abhängigkeit von den sonstigen Prozessparametern erfolgt daher die Zugabe des *o-*Phenylendiamins der Formel (IV) oder eines Gemisches bestehend aus zwei oder mehr *o-*Phenylendiaminen der allgemeinen Formel (IV) in Substanz oder Lösung üblicherweise 0,01-200 min, bevorzugt 0,1-200 min, besonders bevorzugt 20-120 min nach der Vermischung des primären Amins der Formel (V), der Formaldehyd(lösung) und der Protonensäure.

Bevorzugt wird die Temperatur des Reaktionsgemisches in Stufen oder kontinuierlich und ggf. unter Überdruck auf eine Temperatur von 110 °C bis 250 °C, besonders bevorzugt von 110 °C bis 180 °C, ganz besonders bevorzugt von 110 °C bis 160 °C gebracht. Die Verweilzeit wird bevorzugt so gewählt, dass Intermediate mit Aminobenzylanilin-Struktur vollständig umgesetzt sind.

Zur Aufarbeitung des sauren Reaktionsgemisches wird das Reaktionsgemisch üblicherweise mit einer Base neutralisiert. Nach dem Stand der Technik erfolgt die Neutralisation üblicherweise bei Temperaturen von beispielsweise 90 bis 100 °C ohne Zusatz weiterer Substanzen (H.J. Twitchett, Chem. Soc. Rev. 3(2), 223 (1974)). Sie kann aber auch auf einem anderen Temperaturniveau erfolgen, um z.B. den Abbau von störenden Nebenprodukten zu beschleunigen. Als Basen sind beispielsweise geeignet die Hydroxide der Alkali- und Erdalkalielemente. Vorzugsweise kommt wässrige NaOH-Lösung zur Anwendung.

Die zur Neutralisation eingesetzte Base wird bevorzugt in Mengen von größer 100%, besonders bevorzugt 105 bis 120% der für die Neutralisation des eingesetzten sauren Katalysators stöchiometrisch benötigten Menge eingesetzt.

Im Anschluss an die Neutralisation wird in einem Trennbehälter üblicherweise die organische Phase von der wässrigen Phase getrennt. Die nach Abtrennung der wässrigen Phase verbleibende produktenthaltende organische Phase wird anschließend üblicherweise weiteren Aufarbeitungsschritten unterzogen (z.B. Wäsche mit Wasser) und anschließend von überschüssigem Anilin und anderen im Gemisch vorhandenen Stoffen (z.B. weiteren Lösungsmitteln) durch geeignete Verfahren wie z.B. Destillation, Extraktion oder Kristallisation befreit.

Gemäß dem erfindungsgemäßen Verfahren werden Mischungen enthaltend MDA und mehrkernige aromatische Polyamine der Formel (II) und/oder (III), und/oder deren höhere Homologen mit Phosgen in einem inerten organischen Lösungsmittel zu den entsprechenden Polyisocyanatgemischen umgesetzt. Das Verhältnis der eingesetzten Mischung enthaltend MDA und mehrkernige aromatische Polyamine der Formel (II) und/oder (III), und/oder deren höhere Homologen zu Phosgen wird zweckmäßigerweise so bemessen, dass pro Mol NH₂-Gruppe 1 bis 10 Mol, vorzugsweise 1,3 bis 4 Mol Phosgen in der Reaktionsmischung vorliegen.

Als Lösemittel können alle für die Herstellung von Isocyanaten geeigneten Lösemittel eingesetzt werden. Vorzugsweise sind dies inerte aromatische, aliphatische oder alicyclische Kohlenwasserstoffe bzw. deren halogenierte Derivate. Beispiele für solche Lösemittel sind aromatische Verbindungen wie Monochlorbenzol, Dichlorbenzol, beispielsweise o-Dichlorbenzol, Trichlorbenzol, Toluol, Xylole, Naphthalinderivate wie Tetralin oder Decalin, Alkane mit etwa 5 bis etwa 12 C-Atomen wie Hexan, Heptan, Octan, Nonan oder Decan, Cycloalkane wie Cyclohexan, inerte Ester und inerte Ether wie Ethyl- oder Butylacetat, Tetrahydrofuran, Dioxan oder Diphenylether. Insbesondere Anwendung finden als inerte organische Lösungsmittel Monochlorbenzol, Dichlorbenzol oder Mischungen dieser Chlorbenzole. Die Menge an Lösungsmittel wird zweckmäßigerweise so bemessen, dass die resultierende Reaktionsmischung einen Gehalt an aromatischen (Poly)isocyanaten von 2 bis 50 Gew.-%, vorzugsweise zwischen 5 und 30 Gew.-%, bezogen auf das Gesamtgewicht der Reaktionsmischung, aufweist.

In einer alternativen Ausführungsform wird die Umsetzung mit Phosgen in Abwesenheit eines Lösungsmittels durchgeführt.

Die Phosgenierung kann kontinuierlich oder diskontinuierlich in einer oder in mehreren Stufen erfolgen. Wird eine einstufige Umsetzung durchgeführt, so erfolgt diese Umsetzung beispielsweise bei etwa 60 bis 200 °C, vorzugsweise bei etwa 130 bis 180 °C.

In einer weiteren Ausführungsform der Erfindung kann die Umsetzung zweistufig durchgeführt werden. Hierbei wird in einer ersten Stufe die Umsetzung des Phosgens mit der Mischung enthaltend MDA und mehrkernige aromatische Polyamine der Formel (II) und/oder (III), und/oder deren höhere Homologen bevorzugt bei einer Temperatur von 0 bis 140 °C, besonders bevorzugt 20 bis 120 °C, ganz besonders bevorzugt 40 bis 110 °C durchgeführt, wobei für die Reaktion zwischen Amin und Phosgen bevorzugt eine Zeitspanne von 1 min bis 2 h eingeräumt wird. Anschließend wird in einer zweiten Stufe während einer Zeitspanne von 1 min bis 5 h, bevorzugt von 1 min bis 3 h, die Temperatur auf 60 bis 200 °C, bevorzugt 70 bis 170 °C, erhöht.

In einer bevorzugten Ausführungsform der Erfindung wird die Umsetzung in zwei Stufen durchgeführt.

Während der Umsetzung kann ein erhöhter absoluter Druck angelegt werden, der 100 bar oder weniger, bevorzugt 1 bar bis 50 bar, besonders bevorzugt 2 bar bis 40 bar, ganz besonders bevorzugt 3 bar bis 25 bar, beträgt.

Die Umsetzung kann jedoch auch drucklos erfolgen. In einer weiteren Ausführungsform der Erfindung wird daher bei Umgebungsdruck, d.h. bei in der Regel etwa 1 bar absolutem Druck gearbeitet. In einer weiteren Ausführungsform kann zudem bei gegenüber dem Umgebungsdruck reduziertem Druck gearbeitet werden.

Nach beendeter Phosgenierung wird gemäß dem Stand der Technik aus der Reaktionsmischung das überschüssige Phosgen, das inerte organische Lösungsmittel oder Mischungen davon durch Destillation abgetrennt. Die Entfernung des überschüssigen Phosgens erfolgt vorzugsweise bei einer Temperatur von 50 bis 200 °C. Die Entfernung der restlichen Spuren des Lösemittels erfolgt vorzugsweise unter vermindertem Druck. Bevorzugt beträgt der absolute Druck 500 mbar oder weniger, besonders bevorzugt weniger als 100 mbar. Im Allgemeinen werden dabei die verschiedenen Komponenten in der Reihe der Siedepunkte abgetrennt, wobei auch die Abtrennung von Gemischen der verschiedenen Komponenten in einer einzigen Verfahrensstufe möglich ist.

Aus dem gewonnenen Roh-Isocyanat können analog dem für MDI bekannten Stand der Technik Produktgemische, enthaltend zwei- und höherkernige Mono-, Di- und Polyisocyanate, sowie Produkte, enthaltend zweikernige Mono-, Di- und/oder Triisocyanate oder deren Gemische, hergestellt werden. Die Abtrennung dieser Produkte aus dem Roh-MDI kann gemäß dem Stand der Technik, beispielsweise durch Destillation oder Kristallisation, erfolgen. Diese Produkte eignen sich für den Einsatz als farbhelle Rohstoffe für die Polyurethan-Herstellung in Form von Polymeren und Prepolymeren durch Reaktion mit Polyolen.

Die folgenden Beispiele veranschaulichen die vorliegende Erfindung.

### Beispiele

AAV 1: Allgemeine Arbeitsvorschrift für die Herstellung von Mischungen enthaltend MDA und ggf. mehrkernige aromatische Polyamine, die über Methylenbrücken eingebaute *o-*Phenylendiamin-Einheiten aufweisen:
In einem auf 80 °C vorgeheizten Doppelmantelkolben mit mechanischem Rührer werden unter Luftausschluss zunächst 300 g (3,22 mol) Anilin vorgelegt und auf 80 °C erwärmt. Innerhalb von 20 min werden gleichzeitig 631,3 g (6,78 mol) Anilin und 401,5 g (3,75 mol) einer 37,4%igen wässrigen Formaldehydlösung zugegeben. Nachdem das Reaktionsgemisch noch 10 min bei 80 °C nachgerührt worden ist, werden die beiden entstandenen Phasen getrennt. Ca. 300 g der organischen Phase ("Aminal") werden in einem Kolben unter Rühren und Luftausschluss auf 35 °C vorgeheizt. Anschließend werden innerhalb von 30 min bei 35 °C gleichzeitig das restliche "Aminal" und 112,7 g (1 mol) 32,4 %ige Salzsäure zugetropft. Die freiwerdende Exothermie wird mittels externer Kühlung abgefangen. Das Gemisch wird anschließend weitere 90 min bei 35 °C gerührt. Dann wird das Reaktionsgemisch innerhalb von 10 min auf 80 °C erwärmt, eine auf 70 °C vortemperierte 60%ige Lösung von o-TDA (Isomerengemisch bestehend aus ca. 60% 3,4-TDA und 40% 2,3-TDA) in Wasser zugegeben (Mengenangabe siehe Tabelle 1) und das resultierende Reaktionsgemisch für weitere 30 min bei 80 °C gerührt, bevor die Temperatur innerhalb von 30 min auf Rückfluss (ca. 105 °C) erhöht wird. Das Reaktionsgemisch wird weitere 15 h bei dieser Temperatur gerührt. Durch Zugabe von 150 g (1,125 mol) 30%iger wässriger Natronlauge und 400 ml destilliertem Wasser wird das Reaktionsgemisch alkalisiert. Nach 15 min Nachrühren bei ca. 100°C und Phasentrennung wird die organische Phase zweimal bei ca. 100 °C mit 400 ml siedendem destillierten Wasser gewaschen. Die erhaltene organische Phase wird abschließend im Ölpumpenvakuum mittels Destillation von Wasser und überschüssigem Anilin entfernt. Das Produkt verbleibt als Sumpf im Sumpfkolben der Destillation.

Die Analyse des Reaktionsproduktes erfolgte mittels HPLC:
Instrument: HPLC-System Agilent 1100er Serie
Säule: Spherisorb ODS2 / 3µm / 75x4,6mm / MZ-Analysentechnik Art. Nr. 75.4,6.7063.N
Temperatur: 35°C
Injektionsvolumen: 5µL
DAD Signal: 240 nm Bw 12 nm / Referenz 550 nm Bw 40 nm
Eluent A: Acetonitril (Chromasolv / Sigma Aldrich / 34851-2.5L)
Eluent B: demineralisiertes Wasser mit 1,9g Ammoniumacetat pro Liter (p.A. für die HPLC / Sigma-Aldrich / 17836-50g)
Eluent C: Methanol (Gradient / Sigma Aldrich / m.1.06007.2500)
Gradient:

| Zeit | CH₃CN | Wasser | Methanol | Fluss |
|---|---|---|---|---|
| [min] | [%] | [%] | [%] | [mL/min] |
| 0,00 | 6,0 | 74,0 | 20,0 | 1,60 |
| 1,00 | 6,0 | 74,0 | 20,0 | 1,60 |
| 2,00 | 8,0 | 72,0 | 20,0 | 1,60 |
| 2,50 | 8,0 | 72,0 | 20,0 | 1,60 |
| 4,50 | 15,0 | 65,0 | 20,0 | 1,60 |
| 7,50 | 15,0 | 65,0 | 20,0 | 1,60 |
| 8,00 | 20,0 | 60,0 | 20,0 | 1,60 |
| 14,00 | 26,0 | 54,0 | 20,0 | 1,60 |
| 15,00 | 32,0 | 48,0 | 20,0 | 1,60 |
| 21,00 | 32,0 | 48,0 | 20,0 | 1,60 |
| 22,00 | 40,0 | 40,0 | 20,0 | 1,60 |
| 23,00 | 45,0 | 35,0 | 20,0 | 1,60 |
| 24,00 | 55,0 | 25,0 | 20,0 | 1,60 |
| 25,00 | 60,0 | 20,0 | 20,0 | 1,60 |
| 27,00 | 60,0 | 20,0 | 20,0 | 1,60 |
| 27,50 | 32,0 | 48,0 | 20,0 | 1,60 |
| 28,00 | 6,0 | 74,0 | 20,0 | 1,60 |
| 30,00 | 6,0 | 74,0 | 20,0 | 1,60 |

Kalibrierung: Für 4,4'-MDA, 2,4'-MDA und 2,2'-MDA wird eine Mehrpunktkalibrierung mit Reinsubstanzen durchgeführt. Da für die mehrkernigen aromatischen Polyamine (II) und (III) keine geeigneten Reinsubstanzen vorlagen, wurde aufgrund der strukturellen Ähnlichkeit der Responsefaktor für 4,4'-MDA, für die Quantifizierung dieser Verbindungen verwendet. Die Gehaltsangabe in Gewichtsprozent beruht daher auf der Verwendung des Responsefaktors für 4,4'-MDA, für diese Verbindungen. Die Zuordnung der entsprechenden Signale für die mehrkernigen aromatischen Polyamine (II) und (III) erfolgte mittels HPLC-MS.
Probenvorbereitung: Ca. 50-60 mg Probe werden in einen 50 ml Messkolben eingewogen. Der Messkolben wird mit Acetonitril bis zur 50 ml Eichmarke aufgefüllt. Ein Teil dieser Lösung wird in eine HPLC-Septumflasche überführt und für die Analytik verwandt.
AAV 2: Allgemeine Arbeitsvorschrift für die Phosgenierung:

### Ausgangsmaterialien:

- Amingemisch, enthaltend MDA und ggf. mehrkernige aromatische Polyamine, die über Methylenbrücken eingebaute *o*-Phanylendiamin-Einheiten aufweisen, erhalten durch sauer katalysierte Kondensation von Anilin und ggf. o-TDA mit Formaldehyd gemäß der allgemeinen Arbeitsvorschrift AAV 1. Für den Gehalt an Komponenten der Formel (II) und (III), siehe Tabelle 1.
- Chlorbenzol wasserfrei
- Phosgen

15 g des Amingemisches werden in 70 ml Chlorbenzol gelöst, auf 55 °C erwärmt und innerhalb von 10 s unter intensivem Rühren zu einer auf 0 °C temperierten Lösung von 32 g Phosgen in 80 ml Chlorbenzol gegeben. Die Suspension wird unter Durchleiten von Phosgen innerhalb von 45 min auf 100 °C und anschließend während 10 min auf Rückflusstemperatur aufgeheizt. Nach weiteren 10 min bei dieser Temperatur wird das Lösungsmittel unter vermindertem Druck bis zu einer Sumpftemperatur von 100 °C abdestilliert. Das rohe Isocyanat wird anschließend in einer Destillationsapparatur bei einem Druck von 4 bis 6 mbar mittels eines Heißluftgebläses bis zum ersten Produktübergang erhitzt und daraufhin mit kalter Luft innerhalb von 5 bis 10 min auf Raumtemperatur abgekühlt. Von dem so erhaltenen Isocyanat wird 1,0 g in Chlorbenzol gelöst und mit Chlorbenzol zu 50 ml verdünnt. Die Extinktion der so erhaltenen Lösung wird bei den beiden Wellenlängen 430 nm und 520 nm bestimmt. Als Messgerät wird ein Photometer Dr. Lange LICO 300 eingesetzt.

### Vergleichsbeispiel:

Phosgenierung von MDA gemäß AAV 2. Das Amingemisch wurde gemäß AAV 1 durch sauer katalysierte Kondensation von Anilin mit Formaldehyd erhalten und enthielt keine Komponenten der Formel (II) oder (III). Die Ergebnisse der Charakterisierung des eingesetzten Amingemisches und der erhaltenen Polyisoycanatgemische sind in Tabelle 1 zusammengefasst.

### Beispiel 1:

Phosgenierung eines Amingemisches enthaltend MDA und mehrkernige aromatische Polyamine, die über Methylenbrücken eingebaute *o*-Phanylendiamin-Einheiten aufweisen, gemäß AAV 2. Das Amingemisch wurde gemäß AAV 1 durch sauer katalysierte Kondensation von Anilin mit Formaldehyd und 4,7 g (0,039 mol; 0,5 Gew.-% bezogen auf Anilin) erhalten. Das Amingemisch enthielt 0,02 Gew.-% Komponenten der Formel (II) und 0,13 Gew.% Komponenten der Formel (III). Die Ergebnisse der Charakterisierung des eingesetzten Amins und der erhaltenen Polyisoycanatgemische sind in Tabelle 1 zusammengefasst.

### Beispiel 2:

Phosgenierung eines Amingemisches enthaltend MDA und mehrkernige aromatische Polyamine, die über Methylenbrücken eingebaute *o*-Phanylendiamin-Einheiten aufweisen, gemäß AAV 2. Das Amingemisch wurde gemäß AAV 1 durch sauer katalysierte Kondensation von Anilin mit Formaldehyd und 9,3 g (0,076 mol; 1,0 Gew.-% bezogen auf Anilin) erhalten. Das Amingemisch enthielt 0,23 Gew.-% Komponenten der Formel (II) und 0,30 Gew.% Komponenten der Formel (III). Die Ergebnisse der Charakterisierung des eingesetzten Amins und der erhaltenen Polyisoycanatgemische sind in Tabelle 1 zusammengefasst.

**Tabelle 1:**

| | | Vergleichsbeispiel | Beispiel 1 | Beispiel 2 |
|---|---|---|---|---|
| o-TDA | [g] | 0 | 4,7 | 9,3 |
| | [mol] | 0 | 0,039 | 0,076 |
| o-TDA bezogen auf Anilin | [Gew.-%] | 0 | 0,5 | 1 |
| | [mol-%] | 0 | 0,5 | 0,9 |
| | | | | |
| Umsetzung Anilin/o-TDA mit Formaldehyd: | | | | |
| 4,4'-MDA | [Gew.-%] | 50,97 | 52,03 | 50,96 |
| 2,4'-MDA | [Gew.-%] | 4,42 | 4,47 | 4,42 |
| 2,2'-MDA | [Gew.-%] | 0,16 | 0,17 | 0,22 |
| Summe 2-Kern-MDA | [Gew.-%] | 56,2 | 57,0 | 55,9 |
| Komponente (II) | [Gew.-%] | 0 | 0,02 | 0,23 |
| Komponente (III) | [Gew.-%] | 0 | 0,13 | 0,30 |
| | | | | |
| Phosgenierung: | | | | |
| E430 | | 0,111 | 0,127 | 0,130 |
| E520 | | 0,029 | 0,010 | 0,007 |
| E430/E520 | | 4 | 13 | 19 |
| Viskosität | [mPas] | 89 | 77 | 94 |
| NCO | [Gew.-%] | 32,52 | 32,61 | 32,23 |

Beispiele 1 und 2 verdeutlichen gegenüber dem Vergleichsbeispiel, dass bei Verwendung von Mischung enthaltend MDA und mehrkernige aromatische Polyamine der Formel (II) und/oder (III), und/oder deren höhere Homologen, die durch Zusatz von o-TDA zur Reaktion von Anilin und Formaldehyd erhalten werden, das nach Phosgenierung erhaltene Isocyanat bei annähernd gleichem Gelbwert (E430) einen deutlich niedrigeren Grauwert (E520) aufweist. Damit verbunden ist auch ein deutlicher Anstieg des Verhältnisses von Gelbwert zu Grauwert (E430/E520). Damit ist der vorteilhafte Effekt von o-Phenylendiaminstrukturen auf die Farbe des erhaltenen Isocyanates gezeigt.

## Patentansprüche

1. Verfahren zur Herstellung von farbhellen Polyisocyanatgemischen durch Phosgenierung von Mischungen enthaltend Di- und Polyamine der Diphenylmethanreihe (MDA) und mehrkernige aromatische Polyamine der Formel (II) und/oder (III) 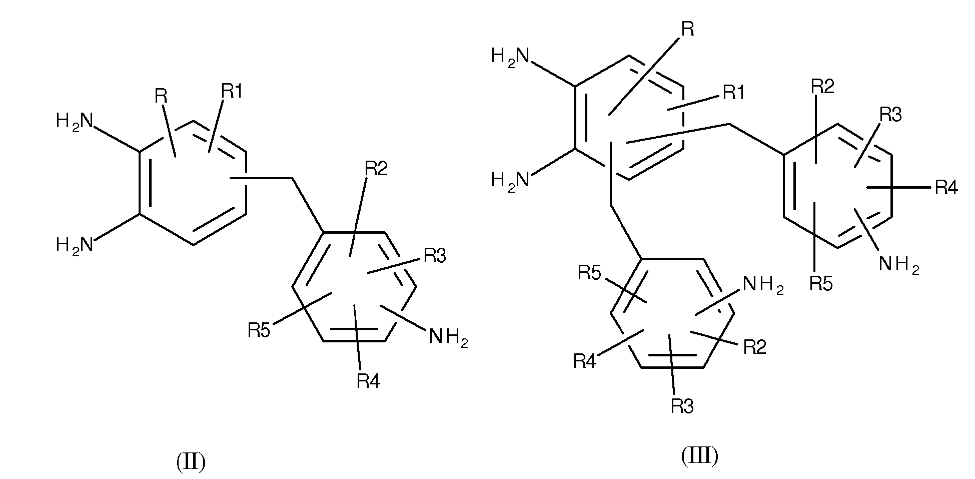 und/oder höhere Homologe der Verbindungen der Formel (II) und/oder (III), wobei
R für Wasserstoff, einen gegebenenfalls substituierten oder Heteroatome enthaltenden gesättigten oder ungesättigten Alkyl-, Cycloalkyl-, Aralkyl- oder Arylrest und
R1 für Wasserstoff, einen gegebenenfalls substituierten oder Heteroatome enthaltenden gesättigten oder ungesättigten Alkyl-, Cycloalkyl-, Aralkyl- oder Arylrest steht,
oder R und R1 direkt miteinander verknüpft sind, so dass sie zusammen mit der Phenylendiaminteilstruktur ein bicyclisches Strukturelement bilden, und
R2, R3, R4 und R5 unabhängig voneinander für Wasserstoff oder einen ggf. substituierten oder Heteroatome enthaltenden gesättigten oder ungesättigten Alkyl-, Cycloalkyl-, Aralkyl- oder Arylrest in *ortho*-, *meta-* oder *para*-Stellung zur an das gleiche aromatische System gebundenen Aminogruppe stehen.

2. Verfahren gemäß Anspruch 1, wobei R und R1 unabhängig voneinander für Wasserstoff oder Methyl stehen und/oder die Reste R2 bis R5 für Wasserstoff stehen.

3. Verfahren gemäß Anspruch 1 oder 2, wobei sich der Rest (H₂N)₂C₆HRR1 in Formel (II) bzw. (H₂N)₂C₆RR1 in Formel (III) von o-Phenylendiamin, 1,2-Diamino-3-methylbenzol oder 1,2-Diamino-4-methylbenzol ableitet.

4. Verfahren gemäß einem der Ansprüche 1 bis 3, wobei der Gehalt der mehrkernigen aromatischen Polyamine (II) und (III), und/oder deren höherer Homologe in den Ausgangsmischungen 0,001 bis 5 Gewichtsprozent bezogen auf das Gesamtgewicht aus MDA, den mehrkernigen aromatischen Polyaminen (II) und (III) und deren höheren Homologen beträgt.

5. Verfahren gemäß einem der Ansprüche 1 bis 4, wobei der Gehalt der mehrkernigen aromatischen Polyamine (II) und (III) in den Ausgangsmischungen 0,001 bis 2 Gewichtsprozent bezogen auf das Gesamtgewicht aus MDA, den mehrkernigen aromatischen Polyaminen (II) und (III) und deren höheren Homologen beträgt.

6. Polyisocyanatmischungen enthaltend Di- und Polyisocyanate der DiphenylmethanReihe (MDI) und mehrkernige aromatische Polyisocyanate, die von den mehrkernigen aromatischen Polyaminen der Formel (II) und (III) 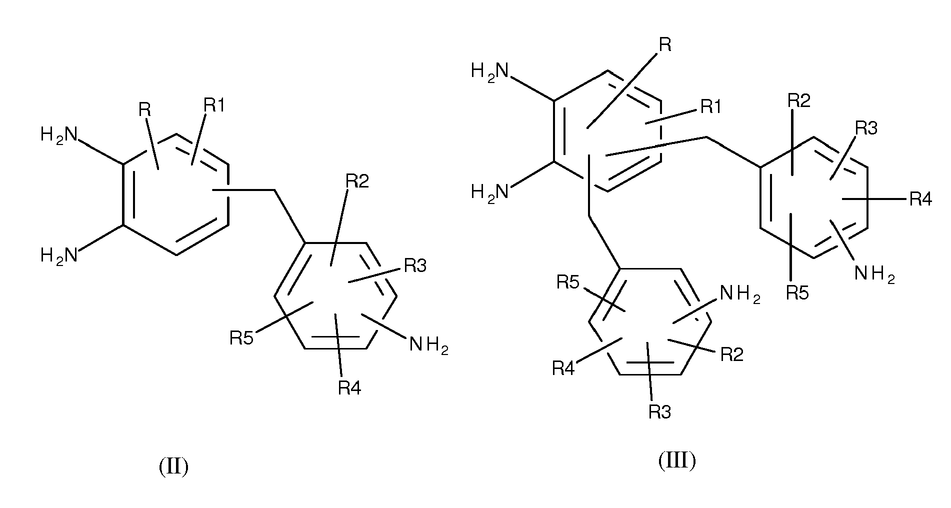 und/oder deren höheren Homologen abgeleitet sind, wobei
R für Wasserstoff, einen gegebenenfalls substituierten oder Heteroatome enthaltenden gesättigten oder ungesättigten Alkyl-, Cycloalkyl-, Aralkyl- oder Arylrest und
R1 für Wasserstoff, einen gegebenenfalls substituierten oder Heteroatome enthaltenden gesättigten oder ungesättigten Alkyl-, Cycloalkyl-, Aralkyl- oder Arylrest steht,
oder R und R1 direkt miteinander verknüpft sind, so dass sie zusammen mit der Phenylendiaminteilstruktur ein bicyclisches Strukturelement bilden, und
R2, R3, R4 und R5 unabhängig voneinander für Wasserstoff oder einen ggf. substituierten oder Heteroatome enthaltenden gesättigten oder ungesättigten Alkyl-, Cycloalkyl-, Aralkyl- oder Arylrest in *ortho*-, *meta-* oder *para*-Stellung stehen.

7. Polyisocyanatmischungen gemäß Anspruch 6, wobei R und R1 unabhängig voneinander für Wasserstoff oder Methyl stehen und/oder die Reste R2 bis R5 für Wasserstoff stehen.

8. Polyisocyanatmischungen gemäß Anspruch 6 oder 7, wobei sich der Rest (H₂N)₂C₆HRR1 in Formel (II) bzw. (H₂N)₂C₆RR1 in Formel (III) von *o*-Phenylendiamin, 1,2-Diamino-3-methylbenzol oder 1,2-Diamino-4-methylbenzol ableitet.

9. Polyisocyanatmischungen gemäß einem der Ansprüche 6 bis 8, wobei der Gehalt der mehrkernigen aromatischen Polyisocyanate, die von den mehrkernigen aromatischen Polyaminen (II) und (III) und/oder deren höheren Homologen abgeleitet sind, in den beanspruchten Polyisocyanatmischungen 0,001 bis 5 Gewichtsprozent bezogen auf das Gesamtgewicht aus MDI und den mehrkernigen aromatischen Polyisocyanaten, die von den genannten mehrkernigen aromatischen Polyaminen (II) und (III) und deren höheren Homologen abgeleitet sind, beträgt.

10. Polyisocyanatmischungen gemäß einem der Ansprüche 6 bis 9, wobei der Gehalt der mehrkernigen aromatischen Polyisocyanate, die von den genannten mehrkernigen aromatischen Polyaminen (II) und (III) in den Ausgangsmischungen abgeleitet sind, 0,001 bis 2 Gewichtsprozent bezogen auf das Gesamtgewicht aus MDI und den mehrkernigen aromatischen Polyisocyanaten, die von den mehrkernigen aromatischen Polyaminen (II) und (III) sowie deren höheren Homologen abgeleitet sind, beträgt.

11. Verwendung der Polyisocyanatmischungen gemäß einem der Ansprüche 6 bis 10 zur Herstellung von Polyurethan-Materialien.

## Claims

1. A method for producing light-coloured polyisocyanate mixtures by phosgenation of mixtures comprising di- and polyamines of the diphenylmethane series (MDA) and polycyclic aromatic polyamines of formula (II) and/or (III) 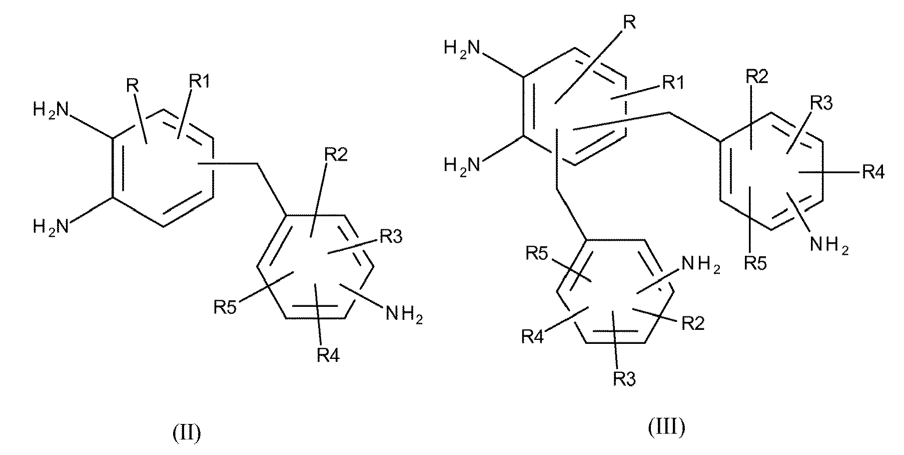 and/or higher homologs of the compounds of formula (II) and/or (III), wherein
R denotes hydrogen, an optionally substituted or heteroatom-comprising, saturated or unsaturated alkyl, cycloalkyl, aralkyl or aryl residue and
R1 denotes hydrogen, an optionally substituted or heteroatom-comprising, saturated or unsaturated alkyl, cycloalkyl, aralkyl or aryl residue,
or R and R1 are directly linked to one another so that they form a bicyclic structural element together with the phenylenediamine partial structure, and R2, R3, R4 and R5 independently of one another denote hydrogen or an optionally substituted or heteroatom-comprising, saturated or unsaturated alkyl, cycloalkyl, aralkyl or aryl residue in *ortho, met* or *para* position to the amino group that is bound to the same aromatic system.

2. The method according to claim 1, wherein R and R1 independently of one another denote hydrogen or methyl and/or the residues R2 to R5 denote hydrogen.

3. The method according to claim 1 or 2, wherein the residue (H₂N)₂C₆HRR1 in formula (II) or (H₂N)₂C₆RR1 in formula (III) is derived from *o-*phenylenediamine, 1,2-diamino-3-methylbenzene or 1,2-diamino-4-methylbenzene.

4. The method according to one of claims 1 to 3, wherein the content of the polycyclic aromatic polyamines (II) and (III) and/or higher homologs thereof in the starting mixtures is 0.001 to 5 wt.%, based on the total weight of MDA, the polycyclic aromatic polyamines (II) and (III) and higher homologs thereof.

5. The method according to one of claims 1 to 4, wherein the content of the polycyclic aromatic polyamines (II) and (III) in the starting mixtures is 0.001 to 2 wt.%, based on the total weight of MDA, the polycyclic aromatic polyamines (II) and (III) and higher homologs thereof.

6. Polyisocyanate mixtures comprising di- and polyisocyanates of the diphenylmethane series (MDI) and polycyclic aromatic polyisocyanates that are derived from the polycyclic aromatic polyamines of formulae (II) and (III) 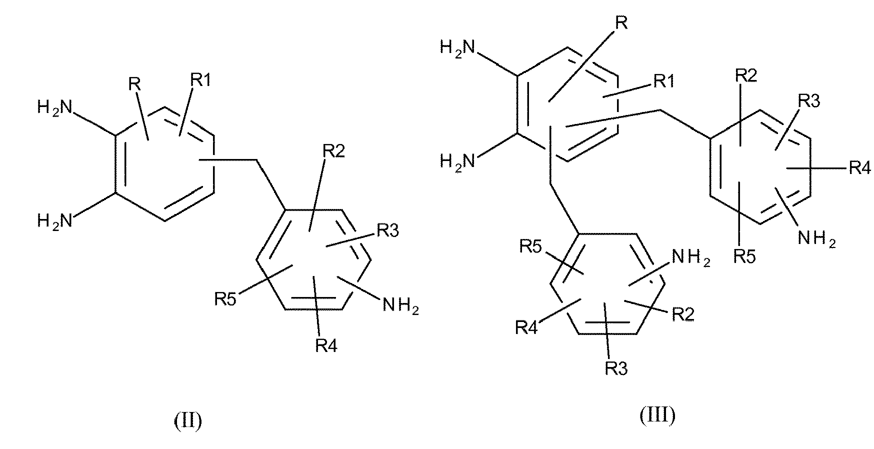 and/or higher homo logs thereof, wherein
R denotes hydrogen, an optionally substituted or heteroatom-comprising, saturated or unsaturated alkyl, cycloalkyl, aralkyl or aryl residue and
R1 denotes hydrogen, an optionally substituted or heteroatom-comprising, saturated or unsaturated alkyl, cycloalkyl, aralkyl or aryl residue,
or R and R1 are directly linked to one another so that they form a bicyclic structural element together with the phenylenediamine partial structure, and
R2, R3, R4 and R5 independently of one another denote hydrogen or an optionally substituted or heteroatom-comprising, saturated or unsaturated alkyl, cycloalkyl, aralkyl or aryl residue in *ortho, meta* or *para* position.

7. The polyisocyanate mixtures according to claim 6, wherein R and R1 independently of one another denote hydrogen or methyl and/or the residues R2 to R5 denote hydrogen.

8. The polyisocyanate mixtures according to claim 6 or 7, wherein the residue (H₂N)₂C₆HRR1 in formula (II) or (H₂N)₂C₆RR1 in formula (III) is derived from o-phenylenediamine, 1,2-diamino-3-methylbenzene or 1,2-diamino-4-methylbenzene.

9. The polyisocyanate mixtures according to one of claims 6 to 8, wherein the content of the polycyclic aromatic polyisocyanates that are derived from the polycyclic aromatic polyamines (II) and (III) and/or higher homologs thereof in the claimed polyisocyanate mixtures is 0.001 to 5 wt.%, based on the total weight of MDI and the polycyclic aromatic polyisocyanates that are derived from the said polycyclic aromatic polyamines (II) and (III) and higher homologs thereof.

10. The polyisocyanate mixtures according to one of claims 6 to 9, wherein the content of the polycyclic aromatic polyisocyanates that are derived from the said polycyclic aromatic polyamines (II) and (III) in the starting mixtures is 0.001 to 2 wt.%, based on the total weight of MDI and the polycyclic aromatic polyisocyanates that are derived from the polycyclic aromatic polyamines (II) and (III) and higher homologs thereof.

11. Use of the polyisocyanate mixtures according to one of claims 6 to 10 for the production of polyurethane materials.

## Revendications

1. Procédé pour la production de mélanges lumineux de polyisocyanates par phosgénation de mélanges contenant des diamines et des polyamines de la série des diphénylméthanes (MDA) et des polyamines aromatiques à plusieurs noyaux de formule (II) et/ou (III) 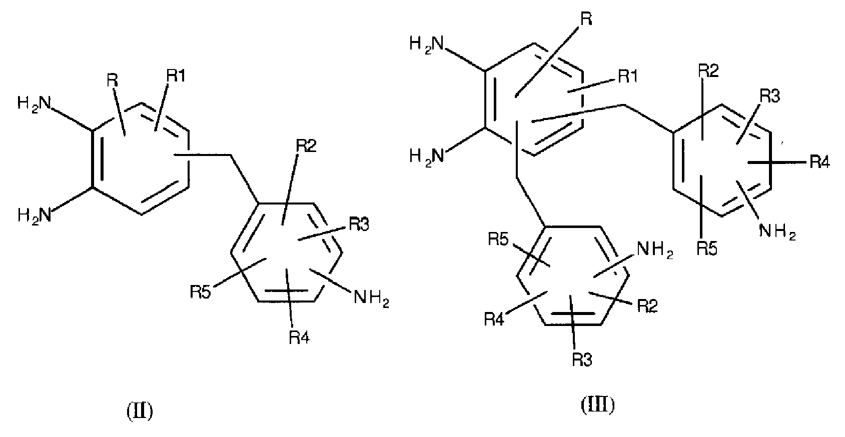 et/ou des homologues supérieurs des composés de formule (II) et/ou (III),
R représentant hydrogène, un radical alkyle, cycloalkyle, aralkyle ou aryle, saturé ou insaturé, le cas échéant substitué ou contenant des hétéroatomes et
R1 représentant hydrogène, un radical alkyle, cycloalkyle, aralkyle ou aryle, saturé ou insaturé, le cas échéant substitué ou contenant des hétéroatomes ou
R et R1 étant liés directement l'un à l'autre de telle sorte qu'ils forment ensemble avec la structure partielle phénylènediamine un élément structural bicyclique, et
R2, R3, R4 et R5 représentant, indépendamment les uns des autres, hydrogène ou
un radical alkyle, cycloalkyle, aralkyle ou aryle, saturé ou insaturé, le cas échéant substitué ou contenant des hétéroatomes, en position ortho, méta ou para par rapport au groupe amino lié au même système aromatique.

2. Procédé selon la revendication 1, R et R1 représentant, indépendamment l'un de l'autre, hydrogène ou méthyle et/ou les radicaux R2 à R5 représentant hydrogène.

3. Procédé selon la revendication 1 ou 2, le radical (H₂N)₂C₆HRR1 dans la formule (II) ou (H₂N)₂C₆RR1 dans la formule (III) étant dérivé d'o-phénylènediamine, de 1,2-diamino-3-méthylbenzène ou de 1,2-diamino-4-méthylbenzène.

4. Procédé selon l'une quelconque des revendications 1 à 3, la teneur en polyamines aromatiques à plusieurs noyaux (II) et (III), et/ou en leurs homologues supérieurs, dans les mélanges de départ étant de 0,001 à 5% en poids par rapport au poids total de MDA, des polyamines aromatiques à plusieurs noyaux (II) et (III) et de leurs homologues supérieurs.

5. Procédé selon l'une quelconque des revendications 1 à 4, la teneur en polyamines aromatiques à plusieurs noyaux (II) et (III) dans les mélanges de départ étant de 0,001 à 2% en poids par rapport au poids total de MDA, des polyamines aromatiques à plusieurs noyaux (II) et (III) et de leurs homologues supérieurs.

6. Mélanges de polyisocyanates contenant des diisocyanates et des polyisocyanates de la série des diphénylméthanes (MDI) et des polyisocyanates aromatiques à plusieurs noyaux, qui sont dérivés des polyamines aromatiques à plusieurs noyaux de formule (II) et (III) 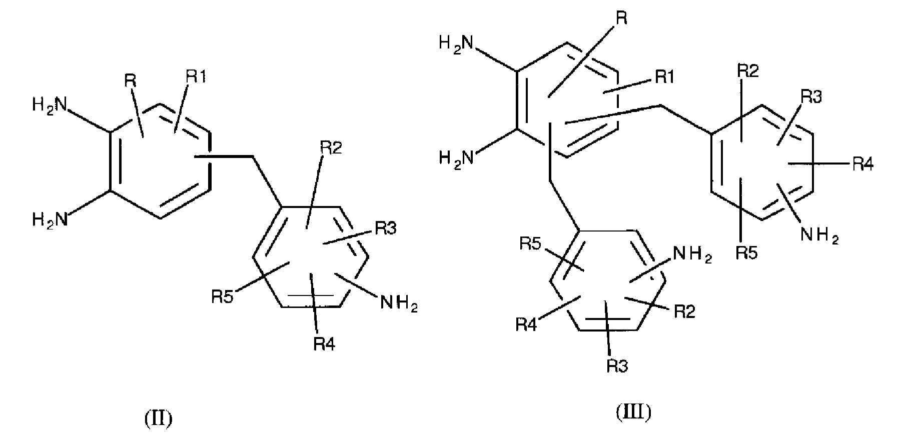 et/ou de leurs homologues supérieurs,
R représentant hydrogène, un radical alkyle, cycloalkyle, aralkyle ou aryle, saturé ou insaturé, le cas échéant substitué ou contenant des hétéroatomes et
R1 représentant hydrogène, un radical alkyle, cycloalkyle, aralkyle ou aryle, saturé ou insaturé, le cas échéant substitué ou contenant des hétéroatomes ou
R et R1 étant liés directement l'un à l'autre de telle sorte qu'ils forment ensemble avec la structure partielle phénylènediamine un élément structural bicyclique, et
R2, R3, R4 et R5 représentant, indépendamment les uns des autres, hydrogène ou un radical alkyle, cycloalkyle, aralkyle ou aryle, saturé ou insaturé, le cas échéant substitué ou contenant des hétéroatomes, en position ortho, méta ou para.

7. Mélanges de polyisocyanates selon la revendication 6, R et R1 représentant, indépendamment l'un de l'autre, hydrogène ou méthyle et/ou les radicaux R2 à R5 représentant hydrogène.

8. Mélanges de polyisocyanates selon la revendication 6 ou 7, le radical (H₂N)₂C₆HRR1 dans la formule (II) ou (H₂NhC₆RR1 dans la formule (III) étant dérivé d'o-phénylènediamine, de 1,2-diamino-3-méthylbenzène ou de 1,2-diamino-4-méthylbenzène.

9. Mélanges de polyisocyanates selon l'une quelconque des revendications 6 à 8, la teneur en polyisocyanates aromatiques à plusieurs noyaux, qui sont dérivés des polyamines aromatiques à plusieurs noyaux (II) et (III), et/ou de leurs homologues supérieurs, dans les mélanges de polyisocyanates revendiqués étant de 0,001 à 5% en poids par rapport au poids total de MDI et des polyisocyanates aromatiques à plusieurs noyaux, qui sont dérivés des polyamines aromatiques à plusieurs noyaux (II) et (III) mentionnées et de leurs homologues supérieurs.

10. Mélanges de polyisocyanates selon l'une quelconque des revendications 6 à 9, la teneur en polyisocyanates aromatiques à plusieurs noyaux, qui sont dérivés des polyamines aromatiques à plusieurs noyaux (II) et (III) mentionnées dans les mélanges de départ, étant de 0,001 à 2% en poids par rapport au poids total de MDI et des polyisocyanates aromatiques à plusieurs noyaux, qui sont dérivés des polyamines aromatiques à plusieurs noyaux (II) et (III) mentionnées ainsi que de leurs homologues supérieurs.

11. Utilisation des mélanges de polyisocyanates selon l'une quelconque des revendications 6 à 10 pour la préparation de matériaux à base de polyuréthanes.
